# EUROPÄISCHE PATENTSCHRIFT

(11) **EP 1 049 536 B1**
(45) Veröffentlichungstag und Bekanntmachung des Hinweises auf die Patenterteilung: **05.03.2003**
(21) Anmeldenummer: 98965659.0
(22) Anmeldetag: 06.11.1998
(51) Int. Cl.: B01J 23/46, B01J 23/56, C07C 17/23, C07C 19/08

(54) **VERFAHREN ZUR HYDRIERENDEN ABREICHERUNG VON FCKW UND HALONEN AUS GASPHASEN**
METHOD FOR HYDROGENATED DEPLETION OF CFC AND HALONS FROM GAS PHASES
PROCEDE D'APPAUVRISSEMENT HYDROGENANT DE CFC ET D'HALONS CONTENUS DANS DES PHASES GAZEUSES

(30) Priorität: 06.11.1997 DE 19750785
(43) Veröffentlichungstag der Anmeldung: 08.11.2000
(73) Patentinhaber: Gesellschaft zur Beseitigung von Umweltschäden mbH, 04936 Schlieben (DE)
(72) Erfinder: FREIBERG, Jürgen, D-16431 Zepernick (DE); ZEHL, Gerald, D-12435 Berlin (DE); MEINKE, Martina, D-14193 Berlin (DE); LÜCKE, Bernhard, D-10179 Berlin (DE)
(74) Vertreter: Gulde, Klaus W., Dipl.-Chem.
(86) Internationale Anmeldenummer: EP9807101
(87) Internationale Veröffentlichungsnummer: WO99024162

(56) Entgegenhaltungen:
- EP-A- 0 737 661
- EP-A- 0 810 192
- WO-A-96/17683
- US-A- 4 918 238
- US-A- 5 134 109
- US-A- 5 136 113
- US-A- 5 426 253

## Beschreibung

Die Erfindung betrifft einen Katalysator und ein Verfahren für die selektive hydrierende Abreicherung von Fluorchlorkohlenwasserstoffen (FCKW) und Halonen aus Gasphasen.

Fluorkohlenwasserstoffe erhalten nach Verboten und restriktivem Einsatz für Fluorchlorkohlenwasserstoffe als ozonschichtneutrale Ersatzstoffe in der Kühlmittelund Treibgasapplikation steigende Bedeutung. Für die technische Herstellung der FKW sind vor allem zwei Verfahrensprinzipien von Interesse, wie dies für die Produktion von Difluormethan CF₂H₂ in den Gleichungen 1 und 2 beispielhaft dargestellt ist. 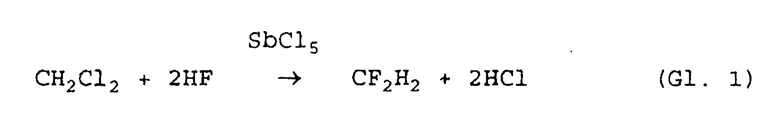 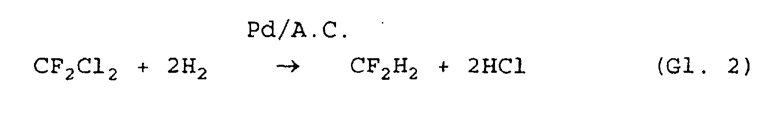 Dies sind:
1. der vollständige Chloraustausch in Chlorkohlenwasserstoffen durch Fluorwasserstoff in Gegenwart vom Friedel-Crafts-Katalysatoren in der Flüssig- und Gasphase (Gl. 1) und
2. die selektive Hydrodechlorierung von FCKW zu FKW bei erhöhten Temperaturen an Trägerkatalysatoren in der Gasphase (Gl. 2).

Nach beiden Verfahrensprinzipien werden Fluorkohlenwasserstoffe produziert, die u.a. FCKW-Anteile aufweisen.

So entsteht nach dem EP 0732314 A1 durch Umsetzung von Methylenchlorid mit Fluorwasserstoff in Gegenwart von Titantetrachlorid bei 120°C im Autoklaven Difluormethan CF₂H₂, das 7% Ausgangsprodukt (CH₂Cl₂) und 10% Chlorfluormethan (CH₂CIF) enthält.

In der WO 9711043 A1 wird CH₂Cl₂ mit überschüssige HF in der Gasphase an Cr0O/Al₂O-Katalysatoren umgesetzt. Das Reaktionsprodukt enthält neben CH₂F₂ Difluormethan größere Mengen an CHCIF₂ Chlordifluormethan und unreagiertes Ausgangsprodukt (CH₂Cl₂). In allen Fällen ist eine aufwendige und verlustreiche Reinigung des Reaktionsproduktes erforderlich.

Durch Hydrodechlorierung von Chlordifluormethan an Pd/Pt-Aktivkohlekatalysatoren in Analogie zu Gl. 2 erhält man nach der WO 96/17683 (1996) unter wirtschaftlichen Bedingungen ein Roh-Difluormethan, das mit 3-7% Ausgangs- Chlordifluormethan und ca. 1% Chlorfluormethan verunreinigt ist. Nach der gleichen hier angegebenen Vorschrift aus Chlorpentafluorethan erzeugtes Pentafluorethan enthält ebenfalls noch 3-5% Ausgangs-FCKW. Auch in diesen Fällen ist eine Reinigungsoperation mittels Druckdestillation teuer und aufwendig.

Diese Verfahren gestatten es demnach nicht, die Fluorkohlenwasserstoff-Produktion so zu führen, daß diese frei von chlorhaltigen Ausgangs-, Zwischen- oder Nebenprodukten sind. Die Beseitigung der ozonschichtgefährdenden und teilweise toxischen (Fluorchlormethan) FCKW/HFCKW ist häufig nur unter aufwendigen Reinigungsoperationen möglich, die die Ökonomie der Verfahren stark belasten.

In der EP 742192 A1 961113 wird ein Pd/AlF₃-Trägerkatalysator beschrieben, der Pentafluorethan von Resten Chlorpentafluorethan in einer Hydrogenolysereaktion bei erhöhter Temperatur zu befreien gestattet. Der Katalysator für diesen Reinigungsprozeß weist jedoch wesentliche Nachteile auf. Er zeigt eine geringe Aktivität und weist eine geringe Langzeitstabilität auf.

Der Erfindung liegt die Aufgabe zugrunde, einen Katalysator und Verfahren zu entwickeln, die unter Schonung weiterer anwesender Gase wie FKW die unerwünschten chlororganischen Anteile weitestgehend zu entfernen gestatte, um so teure Aufarbeitungstechnologien überflüssig zu machen.

Die Aufgabe wird erfindungsgemäß durch die Merkmale des Anspruchs 1 gelöst. Vorteilhafte Ausbildungen der Erfindung sind in den Unteransprüchen angegeben.

In einer bevorzugten Ausführung werden Fluorkohlenwasserstoffe mit chlororganischen Beimengungen in einer heterogenkatalytischen Gasphasenreaktion bei erhöhter Temperatur in Gegenwart von hochaktiven und langzeitstabilen osmium- und/oder rutheniumhaltigen Katalysatoren und von Wasserstoff von diesen chlorierten Produkten befreit. Die Hydrodechlorierungsreaktion verläuft sehr selektiv. Die Fluorkohlenwasserstoffe werden nicht oder nur in geringem Ausmaße angegriffen.
Die auf dem Träger fixierte Aktivkomponentenmischung des erfindungsgemäßen Katalysators enthält mindestens eine Metallzusammensetzung der allgemeinen Formel

OsₐRu_{b}X_{c}Yₐ

in der
- X: Pd, Pt und oder Rh,
- Y: Sc, Ce, Y, Ti, Zr und/oder Hf,
- a: eine Zahl von 0 bis 100,
- b: eine Zahl von 0 bis 100,
- c: eine Zahl von 0 bis 100,
- d: eine Zahl von 0 bis 100,
bedeuten, wobei a + b ≠ 0 sein muß und wobei die untere Grenze an Ru und/oder Os 5 Gew-% bezogen auf den Träger beträgt.

Vorteilhafterweise ist die Abreicherung nicht nur in FKW-haltigen Gasphasen möglich, sondern auch in beliebigen anderen Gasphasen, die keine katalysatorvergiftenden und reaktionsstörenden Bestandteile, wie solche mit oxydierendem oder reduzierendem Verhalten, enthalten. Diese Bestandteile müßten in einen vorgeschalteten Prozeß abgetrennt bzw. entgiftet werden.

Als inerter Katalysatorträger werden Aktivkohle und anorganische Fluoride, Oxide und fluorierte Oxide, wie TiF₄, ZrF₃, AlF₃, Zr(OH)ₓF₃₋ₓ, Al₂0₃, Ti0₂, Zr0₂ eingesetzt. Aus den Oxiden bilden sich während der katalytischen Reaktion chlorid- und fluoridhaltige Oxide. Bevorzugt werden Aktivkohle und Aktivkohle/Zr0₂ verwendet.
Die BET-Oberflächen liegen bei 10 -1500 m²/g, vorzugsweise bei 50 -1000 m²/g. Die Träger sollen frei von Verunreinigungen durch metallische Verbindungen, z.B. eisen- und zinkhaltige Verbindungen sein. Dies kann z.B. bei Aktivkohle und säureunlöslichen Oxiden durch Wäsche mit konzentrierter Salzsäure bei erhöhter Temperatur erreicht werden.

Die Beladung des Trägers mit den Aktivkomponenten kann für Os und/oder Ru von 5 bis 40 Gew.%, für die Metalle der VIII. Nebengruppe von 0 bis 30 % allein oder als Mischung und für die Elemente der III. und IV. Nebengruppe, die sowohl als Träger als auch als Promotoren dienen können, von 0 - 95 Gew.% betragen.

Für die Präparation der Träger mit der Aktivkomponentenmischung werden an sich bekannte Methoden angewendet. Eine typische Herstellungsmethode des erfindungsgemäßen Katalysators erfolgt durch Imprägnation des Trägers mit löslichen Metallverbindungen bzw. hochfeinteiligen Dispersionen von Metallen (Metallsole) und Metallverbindungen oder über die Gasphase. So ist es möglich, eine wäßrige Lösung der Metallsalze, z. B. der Chloride mit anschließender Trocknung des Katalysators, wie sie im Beispiel 1 beschrieben werden, aufzubringen. Die Imprägnation kann stufenweise in Form eines Schalenaufbaus mit einzelnen Bestandteilen der Aktivmetalle und Promotoren oder auch in einem Schritt erfolgen, indem eine Lösung mehrerer Aktivkomponentenverbindungen verwendet wird. Die Aktivkomponentenmischung, die auf dem Träger fixiert wird, enthält in Abhängigkeit von den eingesetzten Ausgangsverbindungen entsprechende Liganden wie Säurereste und Komplexliganden, deren qualitative und quantitative Zusammensetzung sich unter den Reaktionsbedingungen verändern kann.

Der erfindungsgemäße Katalysator ist für die Hydrodehalogenierung von chlororganischen Beimengungen in Fluorkohlenwasserstoffen durch Umsetzung mit Wasserstoff bei erhöhter Temperatur einsetzbar. Ausgangsmaterialien sind die nach den Reaktionsprinzipien der Gl.1 und 2 hergestellten, mit FCKW, HFCKW oder Chlorkohlenwasserstoffen verunreinigten Roh-Fluorkohlenwasserstoffe. Die FKW und die darin enthaltenen chlororganischen Verunreinigungen enthalten 1 bis 6 Kohlenstoffatome, bevorzugt 1 bis 3 C-Atome. Die Konzentration der chlororganischen Verunreinigungen kann zwischen 0.001 bis 30 Vol.%, bevorzugt zwischen 0.01 und 10% betragen. Beispielsweise sind Ausgangsprodukte im Sinne des erfindungsgemäßen Verfahrens:
1. ein nach Gl.1 aus Dichlormethan hergestelltes Difluormethan, das 3 - 5 Vol.% Fluorchlormethan und Reste von Dichlormethan enthält,
2. ein nach Gl. 2 aus Dichlordifluormethan durch Hydrodechlorierung hergestelltes Difluormethan, das 2 - 7 Vol.% Chlordifluormethan, 5 -10 Vol.% Dichlordifluormethan und 0.1-1 Vol.% Chlorfluormethan enthält,
3. ein nach dem Reaktionsprinzip von Gl. 2 aus Chlorpentafluorethan erzeugtes Pentafluorethan, das noch 0.001- 20 Vol.% Chlorpentafluorethan enthält.

Die Reaktion der hydrierenden Abreicherung von chlororganischen Verbindung in Fluorkohlenwasserstoffen in der Gasphase wird bevorzugt bei Temperaturen von 150 - 350°C, bevorzugt bei 200 - 240°C durchgeführt.
Das Reaktionsgas enthält dabei unterstöchiometrische bis mehrfach stöchiometrische Anteile von Wasserstoff, bezogen auf die Hydrodehalogenierung der chlororganischen Verbindungen im jeweiligen Fluorkohlenwasserstoff. Bevorzugt wird ein geringer stöchiometrischer Überschuß bezogen auf die selektive Hydrodehalogenierung der chlororganischen Verbindungen. Überschüssiger, unreagierter Wasserstoff kann in den Prozeß zurückgeführt werden.

Die Hydrodechlorierungsreaktion kann bei atmosphärischem Druck oder unter Druck ausgeführt werden. Limitierend für den Druck sind Kondensation der im Edukt enthaltenen chlororganischen Verbindungen. Es wurde gefunden, daß z.B. ein Edukt aus 5 % Dichlordifluormethan und 2.3 % Chlordifluormethan in Difluormethan bei einem Druck von 20 bar umgesetzt werden kann. Bevorzugt wird die Reaktion bei einem Druck von 1 bis 5 bar durchgeführt.

Die Verweilzeit der Reaktionsgase beträgt gewöhnlich 0.1 bis 80 sec, bevorzugt 2 - 20 sec. Sie ist abhängig von der Art und der Menge der abzureichernden chlororganischen Verbindungen im zu reinigenden Fluorkohlenwasserstoff.

Die Erfindung wird anhand der folgenden Ausführungsbeispiele und Tabelle 1 näher beschrieben.

### Beispiel 1:

100g gebrochene und auf 1.0-1.2 mm Korngröße gesiebte Aktivkohle (z. B. MERCK) mit einer BET-Oberfläche von 1050 m²/g wird 4 Stunden bei 80°C in konzentrierter Salzsäure gerührt, über ein säurefestes Filter von der Waschlösung getrennt, zweimal mit konzentrierter Salzsäure gewaschen und in einem Rundkolben überführt. Die berechneten Mengen OsCl₃, ZrOCl₂ und PdCl₂ werden in 20%iger Salzsäure gelöst und zu der Aktivkohle addiert. Anschließend wird so viel destilliertes Wasser hinzugefügt, daß der Aktivkohleträger gerade mit Flüssigkeit bedeckt ist. Die Slurry wird dann am Rotationsverdampfer bei 50°C zur Trockne abgedampft. Der Katalysator erhält seine Einsatzform nach 10 Stunden Trocknen bei 120°C im Trockenschrank. Der Katalysator hat folgende Zusammensetzung (Gew. %): 5 Teile Os, 2 Teile Pd, 0.5 Teile Zr, 92.5 Teile Aktivkohle ohne Berücksichtigung von Liganden. Die Präparation der in den Tabellen aufgeführten Katalysatormuster erfolgt nach der vorbeschriebenen gleichen Methode durch Variation der als Chloride oder Oxychloride verwendeten Metalle.

### Beispiel 2:

322.3g ZrOCl₂·8H₂O werden in 250ml CH₂Cl₂ aufgeschlämmt und mit einer Lösung von 14.6g OsCl₃ in 50 ml Ethanol unter Rühren versetzt. Nach 1 stündigem Rühren wird das Lösungsmittel am Rotationsverdampfer abgetrieben und der Rückstand 2 h bei 110°C getrocknet. Die trockene krustige Masse wird gebrochen und auf 0.8 -1.5 mm Korngröße ausgesiebt. Der Katalysator erhält seine aktive Form im Verlaufe einer Formierungsphase im Reaktor, in der während der Hydrierreaktion entstandener Fluorwasserstoff zu Bildung fluor- und chlorhaltiger Zirkonverbindungen führt.

### Beispiel 3:

45 cm³ (25.04g) des Katalysators aus Beispiel 1 oder Beispiel 2 (1.0 -1.2 mm Körnung) werden in ein elektrisch über eine LiNO₃/KNO₃-Salzschmelze beheiztes Reaktionsrohr aus V2A-Stahl (40 cm lang,1.8 cm Durchmesser) gefüllt und im Argonstrom von 5 1/h auf 200°C geheizt. Nach 3 Stunden wird der Argonstrom durch das Reaktionsgas (15 1/h) ersetzt. Die Volumenverhältnisse der untersuchten Reaktionsgase ist für die Beispiele in der folgende Tabelle aufgeführt. Die Reaktorabgase passieren eine mit 20 %iger Kalilauge gefüllte Waschflasche zur Absorption der sauren Gasbestandteile und ein mit Natronkalkgranulat versehenes Trockenrohr, bevor sie über eine Probenschleife der gaschromatischen Analyse unterzogen werden. Die Katalysatoreigenschaften werden im Temperaturbereich von 180 - 250°C untersucht.

### Beispiel 4:

Fünf Katalysatoren wurden nach den Präparationsmethoden der Beispiele 1 und 2 hergestellt und bzgl. ihres Leistungsparameters Abreicherung chlororganischer Begleitstoffe bei unterschiedlichen Temperaturen und unter Druck getestet. Beim Vergleich wurde ein Hydrodechlorierungskatalysator nach WO 96/17683 mit 10 % Pd + 2% Pt/AC präpariert und in das Testprogramm integriert. In Tabelle 1 sind die Ergebnisse zusammengefaßt, die zeigen, daß unter Einsatz Osmium und/oder Ruthenium enthaltender Trägerkatalysatoren eine sehr selektive und vollständige Abreicherung des FCKW in den FKW ermöglicht wird. Pd/Pt-Aktivkohle-Hydrodechlorierungskatalysatoren führen nicht zum gewünschten Erfolg.

**Tab.1:**

| Übersicht der untersuchten Katalysatoren und ihrer katalytischen Eigenschaften Feed-Gas A: Difluormethan: Dichlordifluormethan Chlordifluormethan : Chlorfluormethan: H₂ - 9 :1 : 0.5 : 0.05 : 6 Feed-Gas B: Pentafluorethan: Chlorpentafluorethan: H₂ = 9.9 : 0.5 : 2.5 | | | | | |
|---|---|---|---|---|---|
| Nr. | feed-gas | Katalysator | FCKW-Abreicherung auf %bzw. ppm | | |
| | | | 200° C | 250° C | 300° C |
| 1 | A | 10%Os, 2% Pd, 0.5% Zr/A-C. | 5 | 1 | 0 |
| | B | | 1,5 | 0,5 | 0 |
| 2 | A | 5%Os, 2% Ru/A.C. | 6 | 1 | 0 |
| | B | | 3 | 1 | 0 |
| 3 | A | 5%Os/Zr(OH)ₓCl_{y}F_{z} | 6 | 1 | 0 |
| | B | | 3 | 1 | 0 |
| 4 | A | 5%Ru/A.C. | 7 | 2 | 0,5 |
| | B | | 3 | 1 | 0 |
| 5 | A | 10%Os/A.C. | 5 | 1 | 0 |
| | B | | 2 | 0,5 | 0 |
| V | A | 10%Pd, 2%Pt/A.C. | 8 | 7 | 6 |
| | B | | 4 | 3,5 | 3 |

## Patentansprüche

1. Verfahren zur hydrierenden Abreicherung von
a) Dichlordifluormethan (R12) und/oder Chlordifluormethan (R22) und/oder Chlorfluormethan (R31) aus Difluormethan (R32)
oder b) Chlorpentafluorethan (R115) aus Pentafluorethan (R125) in Gegenwart von Wasserstoff in der Gasphase und einer auf einem Träger fixierten Aktivkomponentenmischung, die mindestens eine Metallzusammensetzung der allgemeinen Formel
OSₐRu_{b}X_{c}Y_{d}
enthält, in der
X Pd, Pt und/oder Rh,
Y Sc, Ce, Y, Ti, Zr und/oder Hf,
a eine Zahl von 0 bis 100
b eine Zahl von 0 bis 100
c eine Zahl von 0 bis 100
d eine Zahl von 0 bis 100
bedeuten, wobei a + b ≠ 0 sein muß und
wobei die untere Grenze an Ru und/oder Os 5 Gew-% bezogen auf den Träger beträgt, durchgeführt wird.

2. Verfahren nach Anspruch 1,
**dadurch gekennzeichnet,**
**daß** für X und Y je ein oder mehrere Metalle stehen.

3. Verfahren nach Anspruch 1 oder 2,
**dadurch gekennzeichnet,**
**daß** der Träger frei von Verunreinigungen durch metallische Verbindungen wie eisen- und zinkhaltige Verbindungen ist.

4. Verfahren nach einem der Ansprüche 1 bis 3,
**dadurch gekennzeichnet,**
**daß** der Träger aus Aktivkohle, anorganischen Fluoriden, Oxiden und fluorierten Oxiden, wie TiF₄, ZrF₃, AlF₃, Zr(OH)ₓF₃₋ₓ, Al₂0₃, Ti0₂ und/oder Zr0₂ besteht.

5. Verfahren nach einem der Ansprüche 1 bis 4,
5. Verfahren nach einem Ansprüche 1 bis 4,
**dadurch gekennzeichnet,**
**daß** der Gehalt an Osmium und/oder Ruthenium sowie weiterer Metalle 5% bis 40% Gew.-% bezogen auf den Träger beträgt.

6. Verfahren nach einem der Ansprüche 1 bis 5,
**dadurch gekennzeichnet,**
**daß** der Gewichtsanteil von Osmium und/oder Ruthenium zu den weiteren Elementen 0,1:1 bis 100:1 beträgt.

7. Verfahren nach einem der Ansprüche 1 bis 6,
**dadurch gekennzeichnet,**
**daß** Osmium oder Ruthenium allein oder als Gemisch auf dem Träger fixiert sind.

8. Verfahren nach einem der Ansprüche 1 bis 7,
dadurch gekennzeichent,
daß er als Träger Aktivkohle und als Aktivmetall 5-20% Osmium enthält.

9. Verfahren nach einem der Ansprüche 1 bis 8,
daurch **gekennzeichnet**,
daß er als Träger Aktivkohle und als katalytisch wirksame Metalle 5-20 Gew.-% Osmium und Ruthenium im Verhältnis 10:1 bis 1:10 enthält.

10. Verfahren nach einem der Ansprüche 1 bis 9,
**dadurch gekennzeichnet,**
**daß** die Aktivkomponentenmischung Liganden wie Säurereste und/oder Komplexliganden enthält.

11. Verfahren nach einem der Ansprüche 1 bis 10,
**dadurch gekennzeichnet,**
**daß** die Abreicherung aus für den Katalysator und die katalytischen Reaktionen inerten Gasphasen durchgeführt wird.

12. Verfahren nach einem der Ansprüche 1 bis 11,
**dadurch gekennzeichnet,**
**daß** die Volumenanteile an Dichlordifluormethan, Chlordifluormethan und Chlorfluormethan allein oder in der Summe einzelner oder aller Chlorverbindungen zwischen 0.001-30% beträgt.

13. Verfahren nach einem der Ansprüche 1 bis 12,
**dadurch gekennzeichnet,**
**daß** der Volumenanteil von Chlorpentafluorethan (R115) im Pentafluorethan (R125) zwischen 0.001-20% beträgt.

14. Verfahren nach einem der Ansprüche 1 bis 13,
**dadurch gekennzeichnet,**
**daß** die Reaktion der hydrierenden Abreicherung in der Gasphase bei Temperaturen von 130-400°C durchgeführt wird.

15. Verfahren nach einem der Ansprüche 1 bis 14,
**dadurch gekennzeichnet,**
**daß** die hydrodehalogenierende Abreicherung in der Gasphase bei 150° bis 250° durchgeführt wird.

16. Verfahren nach einem der Ansprüche 1 bis 15,
**dadurch gekennzeichnet,**
**daß** der Wasserstoff in unterstöchiometrischen bis mehrfach stöchiometrischen Anteilen, bezogen auf die FCKW- und/oder Halonhydrierung zu den Fluorkohlenwasserstoffen, eingesetzt wird.

17. Verfahren nach einem der Ansprüche 1 bis 16,
dadurch gekennzeichent,
daß der Wasserstoff in stöchiometrischen bis 3-fachen stöchiometrischen Anteilen, bezogen auf die Fluorkohlenwasserstoff-Hydrierung eingesetzt wird.

18. Verfahren nach einem der Ansprüche 1 bis 17,
**dadurch gekennzeichnet,**
**daß** die Abreicherung im Kreislauf geführt wird, bei Reaktionsprodukte teilweise und/oder vollständig vor Wiedereintritt in den Reaktionsbereich abgetrennt werden.

19. Verfahren nach einem der Ansprüche 1 bis 18,
**dadurch gekennzeichnet,**
**daß** der Gasphase inerte Gase zugesetzt werden.

## Claims

1. The process for the hydrogenated depletion of
a) dichlorodifluoromethane (R12) and/or chlorodifluoromethane (R22) and/or chlorofluoromethane (R31) from difluoromethane (R32) and/or
b) chloropentafluoroethane (R115) from pentachloroethane (R125)
carried out in the presence of hydrogen in the gas phase and of a mixture of active components fixed on a support, which contains at least one metal composition of general formula
OsₐRu_{b}X_{c}Y_{d}
wherein
X represents Pd, Pt and/or Rh;
Y represents Sc, Ce, Y, Ti, Zr and /or Hf;
a represents a number from 0 to 100;
b represents a number from 0 to 100;
c represents a number from 0 to 100;
d represents a number from 0 to 100;
with the requirement that a + b ≠ 0 and that the lower limit of Ru and/or Os is 5 wt.-% with respect to the support.

2. The process according to claim 1, **characterized in that** each X and Y represents one or more metals.

3. The process according to claim 1 or 2, **characterized in that** the support is free of contaminations by metallic compounds, such as compounds containing iron and zinc.

4. The process according to one of claims 1 to 3, **characterized in that** the support consists of active charcoal, inorganic fluorides, oxides and fluorinated oxides, like TiF₄, ZrF₃, AlF₃, Zr(OH)ₓF₃₋ₓ, Al₂O₃, TiO₂, and/or ZrO₂.

5. The process according to one of claims 1 to 4, **characterized in that** it contains osmium and/or ruthenium and other metals from 5 to 40 wt.-% with respect to the support.

6. The process according to one of claims 1 to 5, **characterized in that** the weight ratio of osmium and/or ruthenium to the other elements is in the range from 0.1:1 to 100:1.

7. The process according to one of claims 1 to 6, **characterized in that** osmium or ruthenium are fixed on the support alone or as a mixture.

8. The process according to one of claims 1 to 7, **characterized in that** the catalyst contains active charcoal as a support and 5 to 20 wt.-% osmium as the active metal.

9. The process according to one of claims 1 to 8, **characterized in that** the catalyst contains active charcoal as a support and 5 to 20 wt.-% osmium and ruthenium as the catalytically effective metals in a ratio from 10:1 to 1:10.

10. The process according to one of claims 1 to 9, **characterized in that** the mixture of active components contains ligands such as acid residues and/or complex ligands.

11. The process according to one of claims 1 to 10, **characterized in that** the depletion is carried out in gas phases being inert with respect to the catalyst and the catalytic reactions.

12. The process according to one of claims 1 to 11, **characterized in that** the volume parts of dichlorodifluoromethane, chlorodifluoromethane and chlorofluoromethane alone or in total of some individual or all chlor compounds is in a range from 0.001 to 30%.

13. The process according to one of claims 1 to 12, **characterized in that** the volume part of chloropentafluoroethane (R115) in pentafluoroethane (R125) is in a range from 0.001 to 20%.

14. The process according to one of claims 1 to 13, **characterized in that** the reaction of hydrogenated depletion is carried out in the gas phase at temperatures of from 130 to 400°C.

15. The process according to one of claims 1 to 14. **characterized in that** the hydrogenated depletion is carried out in the gas phase at temperatures of from 150 to 250°C.

16. The process according to one of claims 1 to 15, **characterized in that** hydrogen is used in a range from substoichiometric to multi-stoichiometric percentages in the CFC and/or halon hydrogenation to form fluorohydrocarbons.

17. The process according to one of claims 1 to 16, **characterized in that** hydrogen is used in a range from stoichiometric to threefold stoichiometric percentages in the hydrogenation to form fluorohydrocarbons.

18. The process according to one of claims 1 to 17, **characterized in that** the depletion is conducted in circulation, the reaction products being removed partially and/or completely prior to being capable of re-entering the reaction zone.

19. The process according to one of claims 1 to 18, **characterized in that** inert gases are added to the gaseous phase.

## Revendications

1. Procédé de réglage d'un système de transport pour agents fluidiques, comprenant plusieurs conduites de transport (11) parallèles avec une condition préalable d'un courant total transporté de manière constante, les conduites de transport (11) étant alimentées à partir d'une unité de transport unique (9),
**caractérisé en ce que**
le courant total transporté de manière constante est divisé en courants partiels prédéfinis dans les conduites de transport respectives (11) par une régulation des résistances d'écoulements partiels (1), qui sont déterminées en continu sous la forme de quotient de la valeur de la pression totale et de la valeur du courant partiel, en comparant les quotients déterminés sous forme de valeurs réelles à une valeur de consigne prédéfinie pour chaque conduite de transport (11) et, en fonction de la valeur de la différence de régulation respective entre la valeur réelle et la valeur de consigne, en effectuant une régulation de la résistance d'écoulement partiel respective (1) et donc du courant partiel de l'agent fluidique dans la conduite de transport respective.

2. Procédé selon la revendication 1,
**caractérisé en ce que**
le courant total est divisé en courants partiels absolument identiques.

3. Procédé selon la revendication 1 ou 2,
**caractérisé en ce que**
du côté de l'entrée, une valeur de pression totale du système de transport ainsi que des valeurs de courants partiels sont mesurées directement dans les conduites de transport (11).

4. Procédé selon la revendication 1 ou 2,
**caractérisé en ce que**
du côté de l'entrée, la valeur de pression totale du système de transport ainsi que des valeurs de courants partiels sont mesurées indirectement dans les conduites de transport (11).

5. Procédé selon l'une quelconque des revendications 1 à 4,
**caractérisé en ce que**
la valeur de consigne est déterminée par le rapport d'un produit de la pression totale et du nombre des conduites de transport (11) sur le courant total.

6. Procédé selon l'une quelconque des revendications 1 à 5,
**caractérisé en ce que**
dans le cas de différentes données préalables des courants partiels, la valeur de consigne déterminée selon la revendication 4 est multipliée par un facteur en fonction de la donnée préalable des courants partiels.

7. Dispositif de régulation de courants partiels individuels d'un système de transport pour agents fluidiques avec une condition préalable d'un courant total transporté de manière constante avec plusieurs conduites de transport guidées en parallèle et une unité de transport unique (9),
**caractérisé en ce que**
au moins un dispositif de mesure de la pression totale (10), un distributeur (8), dans chaque conduite de transport (11.1 à 11.8) au moins une unité de mesure des courants partiels (D) et au moins une soupape (7) avec un étranglement (B) ainsi qu'un module d'enregistrement des données, de traitement et de restitution de valeurs de réglages (C) sont prévus, lesquels sont connectés fonctionnellement les uns aux autres par le biais de matériel et/ou de logiciels, et sont appropriés à la formation de quotients de la valeur de pression totale et des valeurs des courants partiels en tant que valeurs réelles, en vue de leur comparaison avec des valeurs de consigne prédéfinies, et à la restitution de valeurs de régulation résultant de ces comparaisons.

8. Dispositif selon la revendication 7,
**caractérisé en ce que**
l'unité de mesure des courants partiels (D) présente un capillaire de mesure (2) et un dispositif de mesure de pression partielle (3).

9. Dispositif selon la revendication 7,
**caractérisé en ce que**
l'unité de mesure des courants partiels (D) présente un capillaire de mesure (2) et deux dispositifs de mesure de pression partielle (3, 12).

10. Dispositif selon la revendication 7, **caractérisé en ce que** l'unité de mesure des courants partiels (D) présente un capillaire de mesure (2) avec un dispositif de mesure de la pression différentielle.

11. Dispositif selon la revendication 7,
**caractérisé en ce que**
l'unité de mesure des courants partiels (D) présente un débitmètre.

12. Dispositif selon l'une quelconque des revendications 7 à 11,
**caractérisé en ce que**
le module d'enregistrement des données, de traitement et de restitution de valeurs de réglage (C) présente une détection de la valeur réelle (4), une partie d'émission de la valeur de consigne (5), une zone de comparaison (A) ainsi qu'un régulateur (6) avec un comportement de régulation proportionnel et/ou intégral et/ou différentiel.

13. Dispositif selon l'une quelconque des revendications 7 à 12,
**caractérisé en ce que**
le dispositif de mesure de la pression totale (10) est disposé du côté de la sortie d'une unité de transport (9).

14. Dispositif selon l'une quelconque des revendications 7 à 13,
**caractérisé en ce que**
les résistances d'écoulement (1) sont disposées du côté de la sortie du distributeur (8).

15. Dispositif selon l'une quelconque des revendications 7 à 14,
**caractérisé en ce que**
l'étranglement (B) de la soupape (7) et l'unité de mesure des courants partiels (D) sont disposés dans une conduite de transport entre le dispositif de mesure de la pression totale (10) et en série par rapport à la résistance d'écoulement (1).

16. Dispositif selon l'une quelconque des revendications 7 à 15,
**caractérisé en ce que**
la soupape (7) avec l'étranglement (B) et l'unité de mesure des courants partiels (D) sont disposées dans une conduite de transport du côté de la sortie de la résistance d'écoulement (1).

17. Dispositif selon l'une quelconque des revendications 7 à 15,
**caractérisé en ce que**
la soupape (7) avec l'étranglement (B) et l'unité de mesure des courants partiels (D) sont disposées du côté de l'entrée de la résistance d'écoulement (1).

18. Dispositif selon l'une quelconque des revendications 7 à 15,
**caractérisé en ce que**
la soupape (7) avec l'étranglement (B) est disposée du côté de la sortie de la résistance d'écoulement (1) et l'unité de mesure des courants partiels (D) est disposée du côté de l'entrée de la résistance d'écoulement (1).

19. Dispositif selon l'une quelconque des revendications 7 à 15,
**caractérisé en ce que**
l'unité de mesure des courants partiels (D) est disposée du côté de la sortie de la résistance d'écoulement (1) et la soupape (7) avec l'étranglement (B) est disposée du côté de l'entrée de la résistance d'écoulement (1).
